# EUROPEAN PATENT SPECIFICATION

(11) **EP 0 758 376 B1**
(45) Date of publication and mention of the grant of the patent: **06.12.2006**
(21) Application number: 95917753.6
(22) Date of filing: 28.04.1995
(51) Int. Cl.: C12N 5/08, C12N 5/06, C12P 21/02, A01K 67/027, A61K 35/39

(54) **IN VITRO GROWTH OF FUNCTIONAL ISLETS OF LANGERHANS AND IN VIVO USES THEREOF**
IN-VITRO WACHSTUM VON LANGERHANS'SCHEN INSELN UND IHRE VERWENDUNGEN
CROISSANCE IN VITRO D'ILOTS FONCTIONNELS DE LANGERHANS ET UTILISATIONS IN VIVO DE CES ILOTS

(30) Priority: 28.04.1994 US 234071
(43) Date of publication of application: 19.02.1997
(73) Proprietor: University of Florida Research Foundation, Inc., Gainesville, FL 32611 (US)
(72) Inventor: PECK, Ammon B., Gainesville, FL 32608 (US); CORNELIUS, Janet G., Gainesville, FL 32606 (US)
(74) Representative: UEXKÜLL & STOLBERG
(86) International application number: PCT/US1995/005303
(87) International publication number: WO 1995/029988

(56) References cited:
- EP-A- 0 363 125
- WO-A-86/01530
- WO-A-93/00441
- WO-A-94/23572
- PROCEEDINGS OF THE NATIONAL ACADEMY OF SCIENCES OF USA, vol. 77, no. 6, June 1980 WASHINGTON US, pages 3519-3523, GAZDAR A.F. ET 'Continuous, clonal, insulin- and somastostatin-secreting cell lines established from a transplantable rat islet cell tumor'
- UPSALA J MED SCI 98 (1). 1993. 39-52. CODEN: UJMSAP ISSN: 0300-9734, KORSGREN O ET AL 'IN-VITRO SCREENING OF PUTATIVE COMPOUNDS INDUCING FETAL PORCINE PANCREATIC BETA-CELL DIFFERENTIATION IMPLICATIONS FOR CELL TRANSPLANTATION IN INSULIN -DEPENDENT DIABETES MELLITUS.'
- ENDOCRINOLOGY, vol. 111, no. 5, November 1982 pages 1568-1575, MCEVOY R.C. ET AL. 'Tissue Culture of Fetal Rat Islets ...'

## Description

### Background of the Invention

Diabetes is a major public health problem. As presented in the 1987 Report of The National Long-Range Plan to Combat Diabetes commissioned by the National Diabetes Advisory Board, six million persons in the United States are know to have diabetes, and an additional 5 million have the disease which has not yet been diagnosed. Each year, more than 500,000 new cases of diabetes are identified. In 1984, diabetes was directly causal in 35,000 American deaths and was a contributing factor in another 95,000.

Ocular complications of diabetes are the leading cause of new cases of legal blindness in people ages 20 to 74 in the United States. The risk for lower extremity amputation is 15 times greater in individuals with diabetes than in individuals without it. Kidney disease is a frequent and serious complication of diabetes. Approximately 30 percent of all new patients in the United States being treated for end-stage renal disease have diabetes. Individuals with diabetes are also at increased risk for periodontal disease. Periodontal infections advance rapidly and lead not only to loss of teeth but also to compromised metabolic function. Women with diabetes risk serious complications of pregnancy. Current statistics suggest that the mortality rates for infants of mothers with diabetes is approximately 7 percent.

Clearly, the economic burden of diabetes is enormous. Each year, patients with diabetes or its complications spend 24 million patient-days in hospitals. A conservative estimate of total annual costs attributable to diabetes is at least $24 billion (American Diabetes Association est., 1988); however, the full economic impact of this disease is even greater because additional medical expenses often are attributed to the specific complications of diabetes rather than to diabetes itself.

Diabetes is a chronic, complex metabolic disease that results in the inability of the body to properly maintain and use carbohydrates, fats, and proteins. It results from the interaction of various hereditary and environmental factors and is characterized by high blood glucose levels caused by a deficiency in insulin production or an impairment of its utilization. Most cases of diabetes fall into two clinical types: Type I, or juvenile-onset, and Type II, or adult-onset. Type I diabetes is often referred to as Insulin Dependent Diabetes, or IDD. Each type has a different prognosis, treatment, and cause.

Approximately 5 to 10 percent of diabetes patients have IDD. IDD is characterized by a partial or complete inability to produce insulin usually due to destruction of the insulin-producing β cells of the pancreatic islets of Langerhans. Patients with IDD would die without daily insulin injections to control their disease.

Few advancements in resolving the pathogenesis of diabetes were made until the mid-1970s when evidence began to accumulate to suggest that Type I IDD had an autoimmune etiopathogenesis. It is now generally accepted that IDD results from a progressive autoimmune response which selectively destroys the insulin-producing β cells of the pancreatic Islets of Langerhans in individuals who are genetically predisposed. Autoimmunity to the β cell in IDD involves both humoral (Baekkeskov *et al.,* 1982; Baekkeskov *et al.,* 1990; Reddy *et al.* 1988; Pontesilli *et al.,* 1987) and cell-mediated (Reddy *et al.* 1988; Pontesilli *et al.,* 1987; Wang *et al.,* 1987) immune mechanisms. Humoral immunity is characterized by the appearance of autoantibodies to β cell membranes (anti-69 kD and islet-cell surface autoantibodies), β cell contents (anti-carboxypeptidase A₁, anti-64 kD and/or anti-GAD autoantibody), and/or β cell secretory products (anti-insulin). While serum does not transfer IDD, anti-β cell autoantibody occurs at a very early age, raising the question of an environmental trigger, possibly involving antigenic mimicry. The presence of cell-mediated immunological reactivity in the natural course of IDD is evidenced by an inflammatory lesion within the pancreatic islets, termed insulitis. Insulitis, in which inflammatory/immune cell infiltrates are clearly visible by histology, has been shown to be comprised of numerous cell types, including T and B lymphocytes, monocytes and natural killer cells (Signore *et al.,* 1989; Jarpe *et al.,* 1991). Adoptive transfer experiments using the NOD (non-obese diabetic) mouse as a model of human IDD have firmly established a primary role for auto-aggressive T lymphocytes in the pathogenesis of IDD (Bendelac, *et al.,* 1987; Miller *et al.,* 1988; Hanafusa *et al.,* 1988; Bendelac *et al.,* 1988). Unfortunately, the mechanisms underlying destruction of the pancreatic β cells remain unknown.

Numerous strategies (e.g., bone marrow replacement, immunosuppressive drugs and autoantigen immunizations) have been investigated as possible means to arrest the immunological attack against the pancreatic β cells. However, for these approaches to be effective, individuals who will eventually develop clinical disease must be identified. Most often, patients are identified too late for effective intervention therapy since the immunological attack has progressed to a point where a large percentage of the β cells have already been destroyed. Because the β cell is thought to be an end-stage differentiated cell, it is believed that the body has little capacity to regenerate new β cells, thus necessitating regular life-long insulin therapy. Recently, one approach to overcome this problem has been islet cell transplantation. Islet cell transplantation has the disadvantage that the islets are allogeneic which, in turn, can invoke an allo-immune response. Thus, there would be major advantages to growing Islets of Langerhans containing functional β cells directly from IDD patients.

WO 93/00441 discloses a method for establishing hormone -secreting cells *in vitro* comprising the step of (a) selecting at least one cell having hormone-secreting potential from a population of similar cells having hormone-secreting potential, (b) placing said cell in an establishing medium which is capable of promoting the viability of said cell for at least 13 days *in vitro,* and (c) maintaining the viability of at least one of said cells and the progeny of said cell for at least 13 days *in vitro.*

Shieh et al., Autoimmunity 1993; 15(2): 123-35 disclose the preparation of single cell suspensions of islet cells from whole islet cells isolated from the pancreas of mice. Pancreata are removed from male or female mice, minced, and the resulting small fragments placed into glass scintillation vials. 275-350 islets are isolated from the animals. Following two washes with Hank's Balanced Salt Solution (HBSS), the fragments are digested for 15 minutes in a 37°C shaking water bath using a solution of Type V collagenase (4 mg/ml) and Type II DNAse (2000 units/ml). The supernatant of this first digestion is transferred to a 60 mm culture Petri dish where the enzyme reaction is stopped with the addition of ice-cold HBSS supplemented to 1% fetal bovine serum (FBS). New enzyme is then added to the remaining undigested pancreatic material. This process is repeated every 5 minutes to final digestion. Individual islet are picked from the digested pancreata under a dissecting scope using a Lang-Levy micropipet and placed in Iscove's modified Dulbecco's Minimum Essential Medium (IMDM). The islets are pelleted in a low speed centrifugation (200 x g for 10 minutes), the supernatant discarded, and the remaining islets resuspended in PBS containing 0.025 % trypsin, 0.02 M EDTA and 2000 units/ml Type II DNAse. The islets are then dispersed to single-cells by gentle pipetting for at least 10 minutes in a 37°C water bath. The reaction is stopped by the addition of ice-cold IMDM supplemented to 1% FBS. The dispersed cells are washed two times with HBSS. Singe cell suspensions of the pancreatic islets are then suspended in a 55% Percoll (d x 1.070) solution and centrifuged 25 minutes at 600 x g. The vast majority of islet cells move to the top of the gradient while any infiltrating cells move to the bottom of the centrifuge tube. Each fraction is collected and washed twice with HBSS.

### Brief Summary of the Invention

The subject invention concerns the discovery that functional islets containing insulin-producing β cells, as well as other islet cell types, can be grown in long-term cultures from single pluripotent stem cells.

The novel methods of the subject invention take advantage of the discovery that pluripotent stem cells exist even in the pancreas of adult individuals. The cells can be cultured in a high amino acid nutrient medium that is supplemented with normal serum which is preferably derived from the same mammalian species which serves as the origin of the islet cells (homologous serum). This culture is then left undisturbed for several weeks to permit establishment of stromal cells. Once this stromal cell layer is mature, cell differentiation can be initiated by refeeding the cell culture with the high amino acid medium supplemented with homologous normal serum plus glucose. After an additional period of growth, functional islets containing cells which produce insulin, glucagon, somatostatin and other endocrine hormones can then be recovered using standard techniques.

It was not previously known or suspected that pancreatic cells could be used to grow new islet cells, including β cells, in culture. The fortuitous discovery of culture techniques for growing islet-like tissue *in vitro* eliminates what had previously been a substantial and long standing barrier to diabetes research. The novel methods and materials described herein will enable a better understanding of the mechanisms of diabetes. Furthermore, the ability to grow islet cells in culture will now make certain therapies for diabetes possible for the first time. For example, in accordance with the subject invention, new cultured islets from diabetic individuals can be implanted in a patient as a way to control or eliminate the patient's need for insulin therapy because the cultured islets and/or islet cells are able to produce insulin *in vivo*. Thus, the subject invention also concerns the use of the *in vitro* grown islets of the subject invention for implantation into a mammalian species for *in vivo* treatment of IDD.

The subject invention also greatly facilitates genetic engineering of islet cells to resist subsequent immunological destruction. For example, the cultured islet cells can be transformed to express a protein or peptide which will inhibit or prevent the destructive immune process. Other useful proteins or peptides may be expressed.

Thus, the ability to grow functioning islets *in vitro* from the pancreatic cells of an individual represents a major technical breakthrough and facilitates the use of new strategies for treating IDD. The discovery that pluripotent stem cells exist in adult pancreas circumvents the need to use fetal tissue as a source of cells.

The subject invention also concerns the islet cells produced *in vitro* according to the methods described herein. These cells can be produced from a mammalian pancreatic cell suspension cultured *in vitro* and can give rise to functional islet cells and islet-like tissue structures.

The subject invention further concerns the *in vitro* growth, propagation and differentiation of a pancreatic stem cell, *i.e.,* a progenitor cell or cells that can give rise to the formation of all of the different types of cells and tissue that make up a normal pancreas. Moreover, the subject invention concerns the *in vivo* use of *in vitro* grown pancreatic stem cells to produce an "ecto-pancreas" organ that exhibits functional, morphological and histological characteristics similar to those observed in a normal pancreas. Thus, the ability to produce a functional "ecto-pancreas" *in vivo* from *in vitro* grown pancreatic cells can be used to treat, reverse or cure a wide variety of pancreatic diseases that are known to result in damage or destruction of the pancreas.

### Brief Summary of the Figures

**Figures 1A through 1D** show cells grown according to the procedures of the subject invention.
**Figure 2** shows an islet grown according to the subject invention.

### Detailed Description of the Invention

According to the subject invention, functional Islets of Langerhans can for the first time be grown in *in vitro* cultures. The techniques of the subject invention result in cell cultures which can produce insulin, glucagon, somatostatin or other endocrine hormones. Other useful proteins may also be produced by, for example, transforming the islet cell with DNA which encodes proteins of interest. The ability to grow these functional cell cultures enables those skilled in the art to carry out procedures which were not previously possible.

The method of the subject invention involves making suspensions of stem cells from the pancreas of a mammal. Preferably, the stem cells would be from the pancreas of a prediabetic mammal. However, it is also contemplated that cells from mammals already showing clinical signs of diabetes can be utilized with the subject invention. The cell suspensions are prepared using standard techniques. The cell suspension is then cultured in a nutrient medium that facilitates the growth of the cells. In a preferred embodiment, the nutrient medium is one which has a high concentration of amino acids. One such medium is known as Click's EHAA medium and is well known and readily available to those skilled in the art. Other equivalent nutrient mediums could be prepared and utilized by those skilled in the art. The medium used to suspend the islet cells is advantageously supplemented with normal serum from the same species of mammal from which the islet cells originate. Thus, in the case of mouse islets the medium is supplemented with normal mouse serum, whereas in the case of human islet cells the medium is supplemented with normal human serum. The preparation of normal serum is well known to those skilled in the art. The concentration of normal serum used with the cell culture method of the subject invention can range from about 0.5% to about 10%, but for mice is preferably about 1%. For human serum, a higher concentration is preferred, for example, about 5%.

The cell suspension prepared in the nutrient medium supplemented with normal serum is then incubated under conditions that facilitate cell growth, preferably at about 35-40° C and, preferably, in about 5% CO₂ atmosphere. This incubation period is, thus, carried out utilizing standard procedures well known to those skilled in the art. The cell culture is then left undisturbed without feeding for at least about 3 weeks. During this time stromal cells proliferate and establish a monolayer which will ultimately give rise to islet cells. The initiation of cellular differentiation can be brought about by refeeding the cultures with Click's EHAA medium supplemented with normal serum as discussed above. Rapid refeeding was found to induce extensive islet foci formation with considerable cell differentiation. Upon histological examination of the cells in the islet-like structures, at least three distinct cell types were identifiable and appeared similar to islet cells prepared from islets of control mice. The time required for cell differentiation to occur within these foci decreased as the frequency of refeeding was increased.

We have been able to propagate and expand islet-producing cultures through the serial transfer of islet-derived stromal cells plus islet foci to new culture flasks. This facilitates generating sufficient numbers of islets as required for use in methods described herein for, for example, reversing the metabolic problems of IDD.

In order to determine whether the islet-like structures and/or islet cells produced *in vitro* according to the subject invention could reverse IDD, the islet-like structures were implanted into NOD mice. Mice that received the islet implants exhibited a reversal of insulin-dependent diabetes, whereas untreated NOD mice showed signs of clinical disease. In addition, no autoimmune pathogenesis was observed during the duration of the implants. Thus, islet implants of the subject invention can be used *in vivo* to treat diabetes in mammals, including humans.

In a preferred embodiment of the subject invention, the progression of diabetes can be slowed or halted by reimplantation of autologous islets engineered to be resistant to specific factors involved in the immunological attack. For example, the islets can be engineered so that they are resistant to cytotoxic T cell-derived interferon-γ. The availability of long-term cultures of whole islets can also be used in investigations into the pathogenesis of IDD, including the cellular recognition of β cells, the mode of islet infiltration, and the immune mechanisms of β cell destruction. Furthermore, this technology will facilitate islet transplantation, autologous islet replacement, and even development of artificial islets. The growth of these cells according to the procedures of the subject invention has great utility in teaching students important aspects relating to cell differentiation and function.

In a further embodiment of the subject invention, pluripotent pancreatic stem cells have been gronw *in vitro* from pancreas cells isolated from a mammal. A surprising discovery using these *in vitro* grown cells in conjunction with the methods of the subject invention was the ability to grow and produce, *in vivo*, an organ that exhibited functional, morphological and histological endocrine and exocrine tissues. The ecto-pancreas (a pancreas-like organ situated at an abnormal site within the body cavity) produced *in vivo* according to the subject invention represents a major scientific discovery and provides a novel means for studying, treating, reversing or curing a number of pancreas-associated pathogenic conditions.

As used herein, the term "growth" refers to the maintenance of the cells in a living state, and may include, but is not limited to, the propagation and/or differentiation of the cells. The term "propagation" refers to an increase in the number of cells present in a culture as a result of cell division.

Following are examples which illustrate procedures, including the best mode, for practicing the invention. These examples should not be construed as limiting. All percentages are by weight and all solvent mixture proportions are by volume unless otherwise noted.

### Example 1 - Culturing of Functional Islets of Langerhans

Single cell suspensions of islet cells were prepared from whole islets isolated from the pancreas of 19-20 week old prediabetic male NOD/UF mice, as detailed elsewhere (Shieh *et al,* 1993). Typically, about 25% of the male mice in a NOD colony will have overt IDD at this age and all will have severe insulitis. The islet cells were resuspended in Click's EHAA medium supplemented with normal mouse serum (NMS) to 1% (Peck and Bach, 1973; Peck and Click, 1973), plated in a 25 cm² tissue culture flask, and incubated at 37° C in a 5% CO₂ atmosphere. At this stage, two outcomes are possible: first, the islet-infiltrating cells may dominate, thus permitting the establishment of immune cell lines, or second, stromal-like cells may dominate, thus allowing the growth of a "nurse cell" monolayer. Growth of stromal-like cell monolayers appeared to result when islet-infiltrating cells were plated simultaneously but in limited numbers. Enrichment of the islet cells with decreased numbers of infiltrating cells can be achieved by gradient separation (Jarpe *et al.,* 1991). Stromal cell cultures, when left undisturbed for 4-5 weeks (i.e., no refeeding) proliferated to cover the entire bottom surface of the culture vessel. From this monolayer of cells, small rounded cells appeared almost as if budding from the stromal cell layer.

Differentiation of the cultures was initiated by refeeding the cultures with Click's EHAA medium supplemented with NMS and a sugar solution comprising glucose or sucrose or other sugar equivalents. Typically, the sugar is glucose. The concentration of glucose can be between about 10 mM to 25 mM, but typically is between 15 and 17 mM. Preferably, the concentration of glucose in the medium is approximately 16 mM. Techniques for refeeding cell cultures *in vitro* are well known in the art and typically involve removing from about 50% to about 90% of the old nutrient medium and adding fresh medium to the culture flask. Rapid refeeding induced the formation of increasing numbers of centers of islet growth (referred to herein as foci) exhibiting cell differentiation. The rate of refeeding can be, for example, at about one week intervals. Preferably, the rate of refeeding is at about 5 to 6 day intervals.

At peak production, as many as 150-200 foci occurred simultaneously in a single 25 cm² tissue culture flask. As the cell proliferation and differentiation proceeded, the organization of the islet took place and the islet even appeared to surround itself in a capsular material. The islets generally grew to a constant size (although several grew to about twice the general size), then detached off of the stromal layers to float in the medium. These free-floating islets tended to break down within 48-72 hours, similar to isolated pancreatic islets cultured under similar conditions.

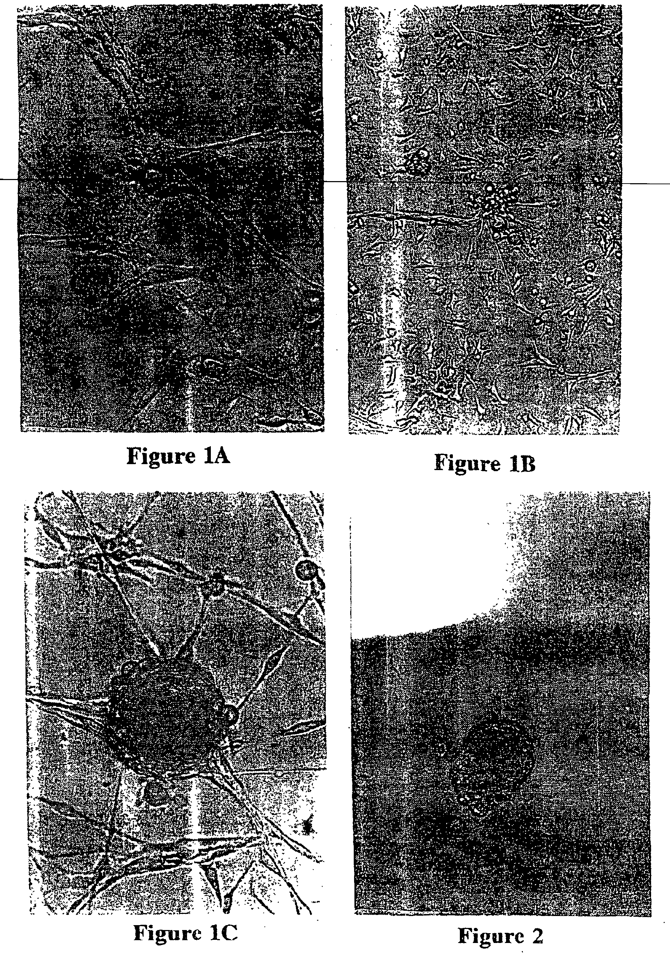

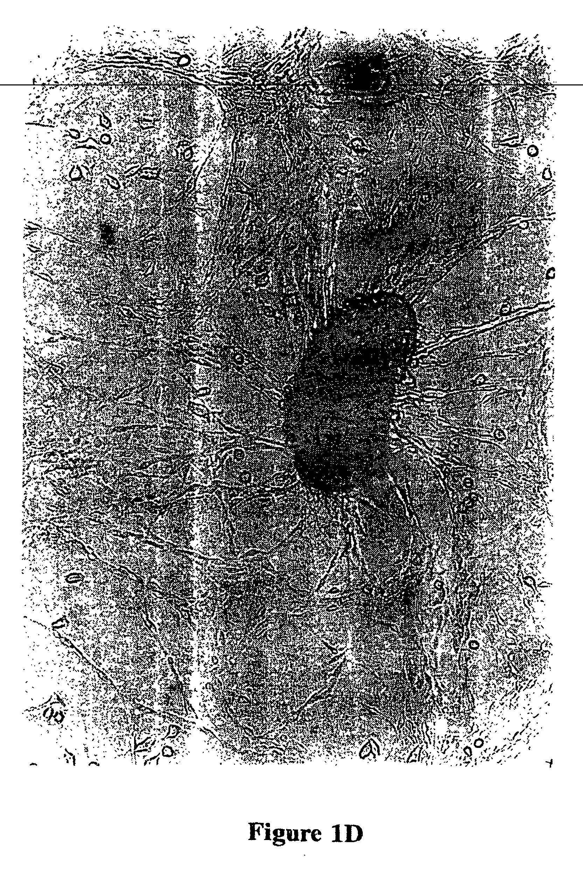

The islet-like structures, collected after natural detachment or removal from the stromal layers using a pasteur pipette, were gently washed in medium, then broken into single cell suspensions by reflux pipetting. Single cell suspensions were prepared by cytocentrifugation, then stained for general morphology and insulin production. The foci contained cells producing the endocrine hormones glucagon (α cells), insulin (β cells) and/or somatostatin (δ cells). Furthermore, the major population of cells stained positive with anti-insulin antibody, indicating the major cell type contained in the cultured islet is an insulin-producing β cell. Figures 1A through 1D show the various cell types which develop during the culture process. Figure 2 shows a well-developed islet obtained after the *in vitro* culture of cells according to the method of the subject invention.

### Example 2 - Culturing of Human Islet Cells

For culturing human islet cells, a procedure similar to that described in Example 1 was utilized. The procedure of the subject invention is particularly advantageous because it is not necessary to utilize fetal cells to initiate the cell culture. In a preferred embodiment, the human cells can be suspended in Click's EHAA medium (or the equivalent thereof) supplemented with normal human serum. Preferably, the concentration of normal human serum used in the medium is about 5%. The cultures should be left undisturbed with no refeeding, preferably for several weeks. After about 4-5 weeks in culture, cell differentiation can be initiated by refeeding the cultures with Click's EHAA medium supplemented with normal human serum and glucose as described in Example 1. Islet-like structures can subsequently be collected and single cell suspensions prepared for further propagation as described in Example 1.

### Example 3 - Implantation of in vitro Grown Islet Cells

To test the efficacy of these *in vitro* generated islet-like structures to reverse the complications of IDD, approximately 150-200 foci plus some stromal cells grown *in vitro* according to the method of the subject invention from pancreatic tissue of NOD mice were dislodged from the tissue culture flask by reflux pipetting. The cells were then implanted beneath the kidney capsule of syngeneic diabetic NOD mice maintained by daily insulin injections. Implantation was accomplished by puncturing the kidney capsule with a hypodermic needle, threading a thin the cortex region. The capillary tube was carefully withdrawn and the puncture site cauterized. The surgical incision of each implanted mouse was clamped until the skin showed signs of healing. The implanted mice were maintained on insulin injections for 4 days at the full daily dosage, and then for 2 days at the half daily dosage, after which the mice were completely weaned from further insulin treatment. Control animals consisted of diabetic NOD mice that did not receive an implant.

Control NOD mice, when removed from daily injections of insulin, showed a rapid onset of overt disease, including lethargy, dyspnea, weight loss, increased blood glucose levels, wasting syndrome, failure of wound healing and death within three weeks. Implanted NOD mice maintained a blood glucose level of about 180 mg/dl (which is slightly above the normal range for mice), showed increased activity, rapid healing of surgical and blood-draw sites, did not develop dyspnea, and remained healthy until killed for histological studies. Similar observations haven been seen with intra-splenic implants.

### Example 4 - In vivo Production of Ecto-Pancreas

Histological examinations of the implant sites in mice that were implanted with the islet cells as described in Example 3 revealed an additional characteristic of the *in vitro* generated islet-forming stem cells. Implanted cells which "leaked" from the implant site of the kidney underwent additional proliferation and differentiation and formed a highly structured ecto-pancreas. At first, the ecto-pancreatic tissue consisted entirely of proliferating exocrine cells which organized into an exocrine pancreas complete with innervating blood vessels. This exocrine pancreas progressed to form islet-like endocrine structures. Thus, the *in vitro* cell cultures produced accoridng ot the methods of the subject invention contain pluripotent pancreatic stem cells capable of regenerating a completely new pancreas. The growth of a pancreas containing both exocrine and endocrine tissue provides new methods for treatment of pancreatic diseases, including pancreatitis and pancreatic cancer.

### References

Eisenbarth, G.S., (1986) *N. Engl. J. Med.* 314:1360.
Cahil, G.F., and H.O. McDevitt (1981) *N. Engl. J. Med* 304:1454.
Todd JA, J.A., *et al.* (1989) *Nature* 338:587.
Prochazka, M., D.V. Serreze, S.M. Worthen, and E.H. Leiter (1989) *Diabetes* 38:1446.
Baekkeskov, S., *et al.,* (1982) *Nature* 298:167.
Baekkeskov, S., *et al.* (1990) *Nature* 347:151.
Reddy, S., NJ. Bibby, and R.B. Elliot (1988) *Diabetologia* 31:322.
Pontesilli, O., P. Carotenuto, L.S. Gazda, P.F. Pratt, and SJ. Prowse (1987) *Clin. Exp. Immunol.* 70:84.
Wang, Y., L. Hao, R.G. Gill, and KJ. Lafferty (1987) *Diabetes* 36:535.
Karjalainen *et al.* (1992) *N. Engl. J. Med.* 327:302.
Serreze, D.V., E.H. Leiter, E.L Kuff, P. Jardieu, and K. Ishizaka (1988) *Diabetes* 37:351
Signore, A, P. Pozzilli, E.A.M. Gale, D. Andreani, and P.C.L. Beverly (1989) *Biabetologia* 32:282
Jarpe, AJ., M. Hickman, J.T. Anderson, W.E. Winter, and A.B. Peck (1991) *Regional Immunol* 3:305
Bendelac, A, C. Carnaud, C. Boitard, and J.F. Bach (1987) *J Exp. Med* 166:823.
Miller, BJ., M.C. Appel, J.J. O'Neil, and L.S. Wicker (1988) *J. ImmunoL* 140:52.
Hanafusa T. *et al.* (1988) *Diabetes* 37:204.
Bendelac A. *et al.* (1988) *J. ImmunoL* 141:2625.
Rossini, A.A., J.P. Mordes, and E.S. Handler (1988) *Diabetes* 37:257.
Nerup, J., *et al.* (1989) *Diabetes Care 11:16.*
Kanazawa, Y., *et al.* (1984) *Diabetologia* 27:113.
Anderson, J.T., J.G. Cornelius, A.J. Jarpe, W.E. Winter and A.B. Peck (1993) *Autoimmunity* 15:113.
Shieh. D.C., J.G. Cornelius, W.E. Winter, and A.B. Peck (1993) *Autoimmunity* 15:123.
Peck, A.B. and F.H. Bach (1973) *J. Immunol. Methods* 3:147.
Peck, A.B. and R.E. Click (1973) *European J. Immunology* 3:382.

## Claims

1. A method for the *in vitro* generation of islet-like structures and islet cells, which comprises culturing pancreatic cells derived from a mammalian species in a nutrient medium supplemented with normal serum, allowing said pancreatic cells to grow undisturbed for at least about 3 weeks until stromal cells proliferate and establish a monolayer of cells which include pancreatic stem cells, and initiating cellular differentiation of the stem cells of the monolayer into functional islet cells and islet-like structures by refeeding the cells of the monolayer with a nutrient medium supplemented with normal serum.

2. A method according to claim 1, wherein the pancreatic cells derived from the mammalian species are islet cells.

3. A method according to any preceding claim, wherein the normal serum is obtained from the mammalian species from which the islet cells were obtained.

4. A method according to claim 3, wherein the pancreatic cells are human islet cells and the serum is normal human serum.

5. A method according to any preceding claim, wherein said nutrient medium comprises a high amino acid nutrient medium.

6. A method according to any preceding claim, wherein the culture medium used to refeed said monolayer of cells further comprises glucose.

7. A method according to any preceding claim, wherein the differentiation is initiated at 4 to 5 weeks of culture growth by refeeding of the cells of the monolayer with the nutrient medium supplemented with homologous normal serum.

8. A method according to any preceding claim, wherein after cell differentiation is initiated by refeeding the monolayer of cells, the cells are refed at about one week intervals.

9. A method for producing an endocrine hormone, which comprises carrying out the method of any one of claims 1 to 8, and recovering said endocrine hormone from the isle-like structures and islet cells.

10. A method according to claim 9, wherein said endocrine hormone is insulin, glucagon or somatostatin.

## Patentansprüche

1. Verfahren zur in-vitro-Erzeugung von inselartigen Strukturen und Inselzellen, bei dem man Pankreaszellen, die aus einer Säugerspezies stammen, in einem Nährmedium kultiviert, das mit Normalserum ergänzt worden ist, man die Pankreaszellen für mindestens etwa 3 Wochen ungestört wachsen läßt, bis Stromazellen proliferieren und eine Monoschicht von Zellen erzeugen, die Pankreasstammzellen einschließen, und man die Zelldifferenzierung der Stammzellen der Monoschicht in funktionale Inselzellen und inselartige Strukturen einleitet, indem man die Zellen der Monoschicht erneut mit einem Nährmedium füttert, das mit Normalserum ergänzt worden ist.

2. Verfahren nach Anspruch 1, bei dem die von der Säugerspezies stammenden Pankreaszellen Inselzellen sind.

3. Verfahren nach einem der vorhergehenden Ansprüche, bei dem das Normalserum von der Säugerspezies erhalten wird, von der die Inselzellen erhalten wurden.

4. Verfahren nach Anspruch 3, bei dem die Pankreaszellen menschliche Inselzellen sind und das Serum menschliches Normalserum ist.

5. Verfahren nach einem der vorhergehenden Ansprüche, bei dem das Nährmedium ein Nährmedium mit hohem Aminosäuregehalt enthält.

6. Verfahren nach einem der vorhergehenden Ansprüche, bei dem das zum erneuten Füttern der Monoschicht von Zellen verwendete Kulturmedium ferner Glucose enthält.

7. Verfahren nach einem der vorhergehenden Ansprüche, bei dem man die Differenzierung nach 4 bis 5 Wochen Kulturwachstum einleitet, indem man die Zellen der Monoschicht erneut mit dem Nährmedium füttert, das mit homologem Normalserum ergänzt worden ist.

8. Verfahren nach einem der vorhergehenden Ansprüche, bei dem man die Zellen in Intervallen von etwa einer Woche erneut füttert, nachdem man die Zelldifferenzierung durch erneutes Füttern der Monoschicht von Zellen eingeleitet hat.

9. Verfahren zur Herstellung eines endokrinen Hormons, bei dem man das Verfahren gemäß einem der Ansprüche 1 bis 8 durchführt und man das endokrine Hormon aus den inselartigen Strukturen und Inselzellen gewinnt.

10. Verfahren nach Anspruch 9, bei dem das endokrine Hormon Insulin, Glukagon oder Somatostatin ist.

## Revendications

1. Procédé de génération *in vitro* de structures en forme d'îlots et de cellules d'îlots, qui comprend les étapes consistant à mettre des cellules pancréatiques provenant d'une espèce mammifère en culture dans un milieu nutritif additionné de sérum normal, laisser lesdites cellules pancréatiques croître sans être perturbées pendant au moins 3 semaines environ jusqu'à ce que des cellules stromales prolifèrent et établissent une monocouche de cellules comprenant des cellules souches pancréatiques, et le déclenchement de la différenciation cellulaire des cellules souches de la monocouche en cellules d'îlots fonctionnelles et en structures en forme d'îlots en réalimentant les cellules de la monocouche avec un milieu nutritif additionné de sérum normal.

2. Procédé selon la revendication 1, dans lequel les cellules pancréatiques provenant de l'espèce mammifère sont des cellules d'îlots.

3. Procédé selon l'une quelconque des revendications précédentes, dans lequel le sérum normal provient de l'espèce mammifère à partir de laquelle les cellules d'îlots ont obtenues.

4. Procédé selon la revendication 3, dans lequel les cellules pancréatiques sont des cellules d'îlots humains et le sérum est du sérum humain normal.

5. Procédé selon l'une quelconque des revendications précédentes, dans lequel ledit milieu nutritif comprend un milieu nutritif à teneur élevée en acides aminés.

6. Procédé selon l'une quelconque des revendications précédentes, dans lequel le milieu de culture utilisé pour réalimenter ladite monocouche de cellules comprend en plus du glucose.

7. Procédé selon l'une quelconque des revendications précédentes, dans lequel la différenciation est déclenchée au bout de 4 à 5 semaines de culture en réalimentant les cellules de la monocouche avec le milieu nutritif additionné de sérum normal homologue.

8. Procédé selon l'une quelconque des revendications précédentes, dans lequel, après que la différenciation cellulaire a été déclenchée en réalimentant la monocouche de cellules, les cellules sont réalimentées à intervalles d'environ une semaine.

9. Procédé de production d'une hormone endocrine, qui comprend la réalisation du procédé selon l'une quelconque des revendications 1 à 8 et la récupération de ladite hormone endocrine à partir des structures en forme d'îlots et des cellules d'îlots.

10. Procédé selon la revendication 9, dans lequel ladite hormone endocrine est l'insuline, le glucagon ou la somatostatine.
